# EUROPEAN PATENT APPLICATION

(11) **EP 4 035 706 A1**
(43) Date of publication of application: **03.08.2022**
(21) Application number: 21154251.9
(22) Date of filing: 29.01.2021
(51) Int. Cl.: A61M 1/28, A61M 5/145

(54) **APPARATUS FOR USE IN PERITONEAL DIALYSIS**

(71) Applicant: Bellco S.r.l., 41037 Mirandola (IT)
(72) Inventor: PUVIANI, Fabrizio, 41037 Modena (IT)
(74) Representative: Maschio, Antonio

(57) **Abstract**

The present invention provides an apparatus for supplying a peritoneal dialysate liquid to a patient, the apparatus comprising a supply bag containing the peritoneal dialysate liquid, the supply bag having an outlet for supply of the liquid from the bag; a first plate and a second plate spaced from and opposed to the first plate, the supply bag being located between the first plate and the second plate, the first and second plates being capable of relative movement to squeeze the bag therebetween to expel dialysate liquid from the bag through the outlet; an electrically actuated mechanism to advance the first plate towards the second plate; and a controller operatively associated with the electrically actuated mechanism and configured to control the movement of the first plate towards the second plate.

## Description

### FIELD OF THE INVENTION

The present invention relates to an apparatus for administering peritoneal dialysis solution to a patient.

### BACKGROUND OF THE INVENTION

Due to disease, insult or other causes, a person's renal system can fail. In renal failure of any cause, there are several physiological derangements. The balance of water, minerals and the excretion of daily metabolic load is no longer possible in renal failure. During renal failure, toxic end products of nitrogen metabolism (urea, creatinine, uric acid, and others) can accumulate in blood and tissues.

Kidney failure and reduced kidney function are conventionally treated with dialysis. Dialysis removes waste, toxins and excess water from the body that would otherwise have been removed by normal functioning kidneys. Dialysis treatment for replacement of kidney functions is life-saving for many patients.

Hemodialysis and peritoneal dialysis are two types of dialysis therapies commonly used to treat loss of kidney function. Hemodialysis treatment utilizes the patient's blood to remove waste, toxins and excess water from the patient. The patient is connected to a hemodialysis machine and the patient's blood is pumped through the machine. Catheters are inserted into the patient's veins and arteries to connect the blood flow to and from the hemodialysis machine. As blood passes through a dialyzer in the hemodialysis machine, the dialyzer removes the waste, toxins and excess water from the patient's blood and returns the blood back to the patient. A large amount of dialysate, for example about 120 liters, is used to dialyze the blood during a single hemodialysis therapy. The spent dialysate is then discarded. Hemodialysis treatment lasts several hours and is generally performed in a treatment center about three or four times per week.

Peritoneal dialysis utilizes a dialysis solution or "dialysate", which is infused into a patient's peritoneal cavity through a catheter implanted in the cavity. The dialysate contacts the patient's peritoneal membrane in the peritoneal cavity. Waste, toxins and excess water pass from the patient's bloodstream through the peritoneal membrane and into the dialysate. The transfer of waste, toxins, and water from the bloodstream into the dialysate occurs due to diffusion and osmosis, since the dialysate is formulated to have a higher osmolality than blood. The spent dialysate is drained from the patient's peritoneal cavity after a suitable interval, which removes the waste, toxins and excess water in the spent dialysate from the patient. This cycle is repeated, typically 3-4 times.

There are various types of peritoneal dialysis therapies, including continuous ambulatory peritoneal dialysis ("CAPD"), automated peritoneal dialysis ("APD") and continuous flow peritoneal dialysis. CAPD is a manual dialysis treatment, in which the patient connects an implanted catheter to a drain and allows a spent dialysate fluid to drain from the peritoneal cavity. The patient then connects the catheter to a bag of fresh dialysate and infuses fresh dialysate through the catheter and into the patient's peritoneal cavity. The fresh dialysate is normally heated to body temperature before infusion, for example on a heating plate. The patient disconnects the catheter from the fresh dialysate bag and allows the dialysate to dwell within the cavity to transfer waste, toxins and excess water from the patient's bloodstream to the dialysate solution. After a dwell period, the patient repeats the manual dialysis procedure.

In CAPD the patient performs several drain, fill, and dwell cycles during the day, for example, about four times per day. Each treatment cycle typically takes about an hour.

Automated peritoneal dialysis ("APD") is similar to CAPD in that the dialysis treatment includes a drain, fill, and dwell cycle. APD machines, however, automatically perform three to four cycles of peritoneal dialysis treatment, typically overnight while the patient sleeps. The APD machines fluidly connect to an implanted catheter. The APD machines also fluidly connect to a source or bag of fresh dialysate and to a fluid drain.

The APD machines pump fresh dialysate from the dialysate source, through the catheter, into the patient's peritoneal cavity and allow the dialysate to dwell within the cavity so that the transfer of waste, toxins and excess water from the patient's bloodstream to the dialysate solution can take place. The APD machines then pump spent dialysate from the peritoneal cavity, though the catheter, to the drain. APD machines are typically computer controlled so that the dialysis treatment occurs automatically when the patient is connected to the dialysis machine, for example, when the patient sleeps. That is, the APD systems automatically and sequentially pump fluid into the peritoneal cavity, allow for a dwell, pump fluid out of the peritoneal cavity and repeat the procedure.

As with the manual process, several drain, fill, and dwell cycles will occur during APD. A "last fill" is typically used at the end of APD, which remains in the peritoneal cavity of the patient when the patient disconnects from the dialysis machine for the day. APD frees the patient from having to manually performing the drain, dwell, and fill steps.

A typical system for CAPD is described in EP-A-499718. Itcomprises a stand which is about 2 meters high. At a first upper level, at the upper end of the stand, there are hooks for hanging bags containing ready-mixed supply solution for peritoneal dialysis. The supply bags are connected via tubes to a heat bag positioned just below the supply bags at a second intermediate level. The heat bag is positioned on a heating surface of a weighing device.

The heat bag is filled, under control of valves, from the supply bags and may have a volume of about 2 litres or slightly more. When the contents in the heat bag has achieved the correct temperature, this is fed by gravity to a catheter terminating in the abdominal cavity of the patient. The catheter and the abdominal cavity are at a third level which is below the second level.

When the dialysis solution has fulfilled its task it is drained to a discharge bag positioned on a fourth level. The discharge bag is attached to a hook arrangement which hangs on the weighing device for the heat bag. In this way the same weight measuring element or load cell is used for weighing the heat bag as well as the discharge bag. The contents of the discharge bag are finally drained either directly to a drain or to collection bags which are situated on a fifth and lowest level. All transport of solution occurs by means of gravity between the five different levels. It is obviously important that the patient is situated at a particular level below the heat bag and above the discharge bag.

A similar cycler system is described in US-A-5 141 492 in which only three levels are used. In this case the input amount is not weighed but only determined by the size of the supply bags. The supply bags are heated directly to the appropriate temperature. The discharge bag is also used for collection and is dimensioned to be sufficiently large.Only the collection bag is weighed.

In order to avoid dependence on gravity for feeding the dialysis solution it has been suggested that pump arrangements be used for this purpose. Such a pump arrangement is disclosed in US-A-4 412 917 in the form of a peristaltic pump for feeding dialysis solution to a patient from a supply container positioned at floor level. A pressure monitoring arrangement ensures that the pressure to the catheter does not exceed a predetermined value. The supply container as well as the collection container are positioned on a weighing device for monitoring the inlet and outlet of dialysis solution to and from the patient.

US-A-4 560 472 discloses a pump arrangement for pumping supply solution from a first level to a heat bag positioned at a higher level. The continued transport of the dialysis solution occurs thereafter by means of gravity. In this way the need to lift the heavy supply bags up to a high level is avoided.

US-A-5 004 459 discloses an even more automated system for administering PD-solution. The apparatus comprises a separate filling pump for filling the abdominal cavity and a separate outlet pump for extraction from the abdominal cavity. Two pressure and/or flow sensors detect and limit the pressure and/or flow for the supply and extraction.

US-A-4 311 587 discloses a supply arrangement for dialysis solution through a sterile filter. The object is to avoid peritonitis by filtering the incoming solution. Since the sterile filter implies a large flow resistance, a higher pressure is required than that which can practically be achieved by means of gravity. Thus a bag containing dialysis solution is placed in a belt under the arm and manual pressure is applied with the arm or elbow to the bag in order to press the dialysis solution through the sterile filter and through the catheter into the abdominal cavity. Extraction occurs with the help of gravity. One-way valves are used in this construction.

WO 90/13795 discloses a pump arrangement intended i. a. for use in connection with peritoneal dialysis. The pump arrangement consists of a chamber divided by an elastic membrane. On one side of the membrane is the solution which is to be pumped and on the other side of the membrane there is gas. Positive and negative pressure is supplied to the chamber with gas. The pumped volume is monitored and measured by measuring the gas volume in the chamber for gas. WO 95/20985 discloses a somewhat similar device for delivering dialysis fluid comprising a fluid-tight container having rigid walls and a bag of dialysis fluid therein. A conduit is provided to conduct dialysis fluid from/to the bag to a patient. The bag is emptied and/or filled by applying vacuum or pressurized gas to the interior of the rigid container to expand or compress the bag therein.

US2015/088054 discloses an automated peritoneal dialysis system that includes a chamber having a disposable unit therein. One wall of the chamber is in the form of a piston that is moved by a stepper motor. Dialysis liquid is drawn into the chamber from a supply bag or from the patient by retraction of the piston. Dialysis fluid is expelled from the chamber by advancing the piston. The disposable unit has an inlet and an outlet, and a system of valves regulates the flow of liquid. The overall system is quite complex.

A need remains for improved peritoneal dialysis systems. For example, a need exists to provide simplified peritoneal dialysis systems that are easier for patients to use and operate. Further, needs exist to provide lower cost peritoneal dialysis systems and peritoneal dialysis systems which are less costly to operate. The systems should be such that supply of the dialysis solution to the patient occurs substantially or entirely without the help of gravity. In this way it is possible for the patient to adopt different positions with respect to the apparatus without affecting the function of the apparatus. Suitably, higher pressure can be used than those which are practically possible with supply by means of gravity, to reduce the supply and extraction times, and suitably the system incorporates means to prevent excessive pressure of dialysate supplied to the patient. Suitably, the flow rates can be adapted to the demands of a specific patient. Suitably, the pump arrangement should be sufficiently silent for night operation.

These and other objectives are achieved by the apparatus according to the present invention.

### SUMMARY OF THE INVENTION

The present invention provides an apparatus for supplying a peritoneal dialysate liquid to a patient, the apparatus comprising: a supply bag containing the peritoneal dialysate liquid, the supply bag having an outlet for supply of the liquid from the bag; a first plate; and a second plate, the supply bag being located between the first plate and the second plate, the first and second plates being capable of relative movement to squeeze the bag therebetween to expel dialysate liquid from the bag through the outlet; an electrically actuated mechanism to advance the first plate towards the second plate; and a controller operatively associated with the electrically actuated mechanism and configured to control the movement of the first plate towards the second plate.

### BRIEF DESCRIPTION OF THE DRAWINGS

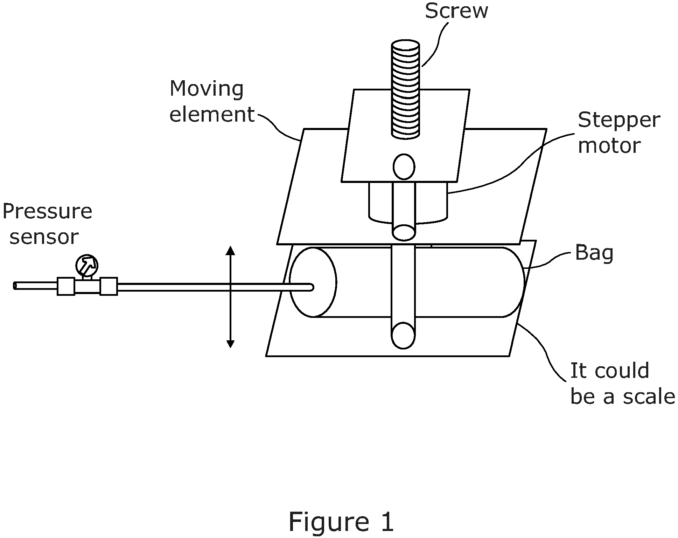

Figure 1 shows an illustration of the invention.

### DETAILED DESCRIPTION OF THE INVENTION

The invention provides an apparatus for supplying a peritoneal dialysate liquid to a patient, the apparatus comprising: a supply bag containing the peritoneal dialysate liquid, the supply bag having an outlet for supply of the liquid from the bag; a first plate; and a second plate spaced from and opposed to the first plate, the supply bag being located between the first plate and the second plate, the first and second plates being capable of relative movement to squeeze the bag therebetween to expel dialysate liquid from the bag through the outlet; an electrically actuated mechanism to advance the first plate towards the second plate; and a controller operatively associated with the electrically actuated mechanism and configured to control the movement of the first plate towards the second plate.

The term "supply bag" herein refers to a primary container of flexible sheet material containing a sterile dialysate for administration to a human patient. The supply bag is suitably sealed before use by a frangible seal. In other embodiments, the outlet may be alternatively or additionally be sealed by a clamp before use. In embodiments, the supply bag has been produced by reconstituting sterile dry components within the bag with sterile water immediately before use.

The term "outlet" herein refers to a tubular outlet having a first end opening into the internal volume of the supply bag and a second end extending outside the supply bag for escape of liquid from the supply bag. The outlet may be integrally formed with the body of the supply bag, or it may be a tube bonded thereto. The supply bag suitably has only one outlet, and suitably no inlet. The amount of dialysate in the supply bag is suitably from about 500 ml to about 2000ml. In embodiments, the supply bag outlet is equipped with a connector element, such as a bard-type connector, for liquid-tight connection to a catheter tube or catheter tube manifold.

The term "dialysate liquid" refers to a sterile aqueous liquid for administration to a patient when performing peritoneal dialysis. The liquid is suitably formulated to contain at least a buffer such as sodium lactate and an osmolality-increasing agent such as glucose in appropriate amounts for dialysis.

The first and second plates are suitably formed of substantially rigid material, optionally coated by a resilient material. The plates are sized and shaped to substantially or completely cover the supply bag when the supply bag has been squeezed-flat, thereby ensuring that all of the liquid in the supply bag is dispensed. For example, the plates may be square plates having sides of from about 10cm to about 30cm. The first and second plates suitably have substantially congruent surfaces, for example parallel flat surfaces, so that the supply bag can be squeezed to expel all of the liquid therefrom. In embodiments, the surfaces of the first and second plates may be profiled, for example with complementary channels, to direct liquid inside the bag towards the outlet.

The first and second plates are mounted in spaced and opposed relationship and configured to be moved towards or apart from one another to squeeze or release the supply bag. For example, the apparatus may comprise a plurality of slide rods fixedly attached around the periphery of the first plate substantially perpendicular to the first plate, and the second plate may define a plurality of apertures correspondingly spaced near the periphery thereof, the slide rods being slidably received in the apertures.

The electrically actuated mechanism to advance the first plate towards the second plate may be any suitable mechanism. In embodiments, the mechanism allows precise control of the relative positions of the first and second plates. For example, the mechanism may comprise a motor coupled to a bolt that is in threaded engagement with one of the plates and in longitudinally fixed engagement with the other plate. The motor could be a stepper motor, or it may incorporate a position sensor such as a Hall effect sensor. The position information correlates to the amount of liquid remaining inside the supply bag, and can therefore be used to control the amount and/or the volumetric rate of liquid expelled from the bag.

In embodiments, the mechanism may alternatively or additionally allow precise control of the force applied between the plates. For example, the motor in the above configurations could include a torque sensor. In any event, the motor and the above sensors therein are suitably operatively connected to the controller, which suitably controls the motor in response *inter alia* to the inputs from the sensors.

The apparatus delivers the peritoneal dialysate by mechanically squeezing the supply bag to expel the liquid under pressure. This eliminates any need for gravity feed of the liquid, thereby permitting greater freedom of movement to the patient. The force applied to the bag can be regulated to regulate the pressure of the liquid at the outlet.

Suitably, the apparatus further comprises a strain gauge configured to measure a force applied between the first and second plates, the strain gauge being operatively associated with the controller. The strain gauge may be a torque meter associated with a motor driving the mechanism as discussed above. Alternatively or additionally, the strain gauge may directly measure a force being applied to one or both of the plates by the mechanism.

The force applied between the first and second plates corresponds substantially to the total squeezing force being applied to the supply bag. This force is related to the pressure being applied to the liquid inside the bag, and hence to the pressure of the liquid being expelled from the outlet.

Suitably, the controller is configured to halt or reverse the approximation of the first and second plates if the force applied between the first and second plates exceeds a predetermined maximum value. This can be used to avoid over-pressurization of the expelled liquid, and to shut down the apparatus if there is a blockage between the plates or when the supply bag is empty.

Alternatively or additionally, the apparatus may further comprise a pressure gauge configured to measure a pressure of the dialysate liquid in the outlet, the pressure gauge being operatively associated with the controller. The pressure gauge is suitably in fluid communication with the liquid in the outlet. It may be directly in fluid communication with the liquid inside the bag, or directly in fluid communication with the inlet, or it may be located in a tubing downstream of the outlet. The pressure gauge provides an electrical output that may be communicated to the controller.

Suitably, the controller is configured to halt or reverse the advancement of the first plate towards the second plate if the pressure of the dialysate liquid in the outlet exceeds a predetermined maximum value. This prevents excessive output pressure that could harm the patient.

It is conventional to heat dialysate liquid before introducing it into a patient. The apparatus according to the present invention may comprise heating means, such as a heating bag, downstream from the outlet. Alternatively, in embodiments, one or both of the plates may further comprise a heater to heat the dialysate liquid expelled from the supply bag. In these embodiments, the heater and associated temperature sensor(s) may be operatively associated with the controller to control the final temperature of the dispensed liquid.

The apparatus of the invention may further comprise a weighing device configured to determine the weight of the dialysate liquid remaining in the supply bag, wherein the weighing device is operatively associated with the controller. The controller may be programmed to dispense a predetermined weight of liquid. For example, the apparatus as herein before described may be supported on a weighing scale, or it may hang from a weighing scale that measures the total weight of the bag, plates, etc., from which changes in weight are due to expulsion of dialysate liquid.

The apparatus of the present invention suitably further comprises a tube or tube-set for connecting the outlet of the supply bag to a catheter entering the body of a patient. The tube or tube-set is suitably releasably connectable to the supply bag, and further comprises an end piece for releasable connection of the tube to the catheter.

## Claims

1. An apparatus for supplying a peritoneal dialysate liquid to a patient, the apparatus comprising:
a supply bag containing the peritoneal dialysate liquid, the supply bag having an outlet for supply of the liquid from the bag;
a first plate; and
a second plate spaced from and opposed to the first plate, the supply bag being located between the first plate and the second plate, the first and second plates being capable of relative movement to squeeze the bag therebetween to expel dialysate liquid from the bag through the outlet;
an electrically actuated mechanism to advance the first plate towards the second plate; and
a controller operatively associated with the electrically actuated mechanism and configured to control the movement of the first plate towards the second plate.

2. An apparatus according to claim 1, further comprising a strain gauge configured to measure a force applied between the first and second plates, the strain gauge being operatively associated with the controller.

3. An apparatus according to claim 2, wherein the controller is configured to halt or reverse the advancement of the first plate towards the second plate if the force applied between the first and second plates exceeds a predetermined maximum value.

4. An apparatus according to any preceding claim, further comprising a pressure gauge configured to measure a pressure of the dialysate liquid in the outlet, the pressure gauge being operatively associated with the controller.

5. An apparatus according to claim 4, wherein the controller is configured to halt or reverse the advancement of the first plate towards the second plate if the pressure of the dialysate liquid in the outlet exceeds a predetermined maximum value.

6. An apparatus according to any preceding claim, further comprising a heater device to heat the dialysate liquid expelled from the supply bag.

7. An apparatus according to claim 6, wherein at least one of the first and second plates is a heater plate.

8. An apparatus according to any preceding claim, further comprising a weighing device configured to determine the weight of the dialysate liquid remaining in the supply bag, wherein the weighing device is operatively associated with the controller.

9. An apparatus according to any preceding claim, wherein the electrically actuated mechanism includes a stepper motor coupled to a screw.
